# EUROPEAN PATENT SPECIFICATION

(11) **EP 1 048 723 B1**
(45) Date of publication and mention of the grant of the patent: **14.04.2004**
(21) Application number: 99938529.7
(22) Date of filing: 19.08.1999
(51) Int. Cl.: C12M 1/00, C12Q 1/68, G01N 35/00, B01J 19/00, G06F 19/00

(54) **BIOCHIP AND METHOD OF USING BIOCHIP**
BIOCHIP UND VERFAHREN ZUR VERWENDUNG DES BIOCHIPS
BIOPUCE ET PROCEDE D'UTILISATION DE LA BIOPUCE

(30) Priority: 09.09.1998 JP 25528898
(43) Date of publication of application: 02.11.2000
(73) Proprietor: Hitachi Software Engineering Co., Ltd., Yokohama-shi, Kanagawa 231-8475 (JP)
(72) Inventor: YOSHII, Jyunji, Hitachi Software Eng., Ltd, Yokohama-shi, Kanagawa 231-8475 (JP); SHIMODA, Masafumi, Hitachi Software Eng. Co., Ltd, Yokohama-shi, Kanagawa 231-8475 (JP); YAMAMOTO, Kenji, Hitachi Software Eng. Co., Ltd, Yokohama-shi, Kanagawa 231-8475 (JP); WATANABE, Toshimasa, Hitachi Software Eng. Co. Ltd, Yokohama-shi, Kanagawa 231-8475 (JP)
(74) Representative: Liesegang, Roland, Dr.-Ing.
(86) International application number: PCT/JP1999/004459
(87) International publication number: WO 2000/014197

(56) References cited:
- EP-A- 0 665 293
- EP-A- 0 695 941
- WO-A-96/05488
- DE-A- 4 310 169
- DE-A- 19 731 479
- JP-A- 4 505 763
- JP-A- 5 026 881
- JP-A- 6 507 498
- US-A- 5 677 197
- US-A- 5 736 332
- US-A- 5 968 728
- VIVIAN G. CHEUNG ET AL.: 'Making and reading microarrays' NATURE GENETICS SUPPLEMENT vol. 21, January 1999, pages 15 - 19, XP002923682
- OSAMU KOBAYASHI: 'Combinatorial synthesis: new technology of synthesizing various chemical compounds by using combination' MODERN CHEMISTRY vol. 304, July 1996, pages 26 - 33, XP002923683

## Description

### TECHNICAL FIELD

The present invention relates to a biochip having a plurality of biopolymer spots arranged thereon such as probe DNAs that specifically hybridize with a specific DNA or protein.

### BACKGROUND ART

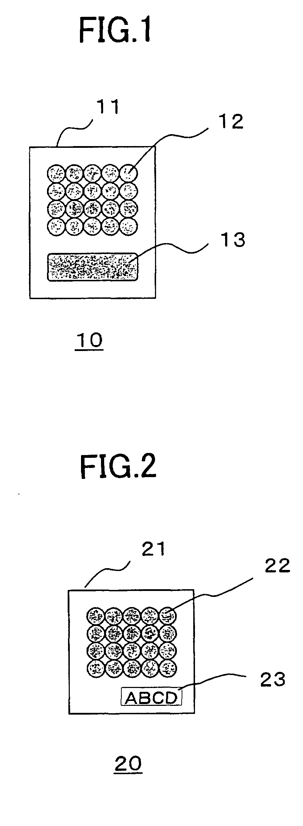

In the biochemistry research of genes or the like, conventionally and presently, biochips have been employed which are made from an immobilization support such as glass or a nylon or nitrocellurose membrane spotted with biopolymers such as DNA or protein in a high-dense pattern. Figure 2 is a schematic view of a conventional biochip. The biochip 20 is provided with an immobilization substrate 21 whose surface is spotted with various types of DNAs 22 in a predetermined pattern. Since the shapes of biochips 20 are identical, there is no way of identifying each biochip by its appearance, nor can the types of the spotted DNAs be identified. Therefore, biochips have been managed by writing an identification number 23 or by providing a barcode on the corner of the biochip 20. In this case, the biochips are managed by leaving a note of information of what kind of DNAs are spotted on which locations on a biochip with a particular identification number or barcode.

According to the above-described method, the biochip may be identified by using two pieces of information written on separate components. However since academic information such as what kinds of nucleic acid sequences of DNAs are spotted on the chip has to be managed as another piece of information, it is often mistakenly used during the experiment.

The present invention aims at solving such problems of conventional technique, and provides a biochip whose information can unitarily be managed and provides a method for using the biochip.

### DISCLOSURE OF THE INVENTION

According to the present invention, the above-described aim is accomplished by integrating a memory into a biochip so as to store information such as types, amounts and spotting locations of spotted DNAs. This biochip allows the entire information of the biochip (e.g., what kind of DNAs are spotted on which locations of the biochip, and when, by whom and in what kind of experiment the biochip was used) to unitarily be managed.

Specifically, the biochip of the invention is provided with a surface on which a plurality of biopolymers are spotted in a predetermined pattern, and a storage medium for storing information of the spotted biopolymers.

The biochip according to the present invention is provided with a surface spotted with a plurality of biopolymers such as DNAs and proteins in a predetermined pattern, and a storage medium for storing information of the biopolymers. The DNAs that are spotted on the biochip may be a plurality of probe DNAs or DNAs from individuals, which are used for genetic diagnosis or gene expression analysis.

The component with the biopolymer-spotted surface and the storage medium may be detachable from each other or they may be formed integrally. Preferably, the storage medium is a semiconductor memory capable of reading/writing information in a non-contact state.

The storage medium may be stored with information of the spotting locations on the surface of the biochip in relation to information of the biopolymers spotted on the spotting locations. The information of the spotting locations may be represented, for example, by sequential numbers of the patterned spot, or coordinates representing the spotting locations. The information of the biopolymers to be stored may be, for example, information of nucleotide sequences of the DNAs, genetic diseases relative to the DNAs, genes relative to the DNAs, and the amounts of spots.

The biochip having the storage medium of the invention is used as follows. A plurality of biopolymers are spotted onto the surface of the biochip in a predetermined pattern. The storage medium is written with information of the spotting locations as well as information of the biopolymers (e.g., DNA nucleotide sequences) spotted on the spotting locations. The information may be written to the storage medium every time when the biopolymers are spotted, or may be written afterwards at one time.

The biochip of the invention whose surface is spotted with a plurality of biopolymers in a predetermined pattern, and which is provided with a storage medium for storing information of the spotting locations in relation to the information of the biopolymers spotted on the spotting locations, is used as follows. A sample is allowed to contact with the biochip; a spotting location that hybridized with the sample is detected by utilizing fluorescence from a fluorescent label; the database in the storage medium is searched for information of the sample-hybridized biopolymer based on the detected hybridized spotting location; and the result is displayed.

### BRIEF DESCRIPTION OF THE DRAWINGS

Figure **1** is a schematic view showing an exemplary biochip of the invention;
Figure **2** is a schematic view showing a conventional biochip;
Figure **3** is a schematic view showing another exemplary biochip of the invention;
Figure **4** is a schematic view showing yet another exemplary biochip of the invention;
Figure **5** is a schematic diagram showing an example of information to be stored in the storage medium attached to the biochip;
Figure **6** is a view for illustrating the process for producing the biochip;
Figure **7** is a view for illustrating a step for inspecting the biochip;
Figure **8** is a view for illustrating hybridization process using the biochip of the invention;
Figure **9** is a view for illustrating process for reading the biochip of the invention;
Figure **10** is a view showing an example of a screen for displaying results obtained with the biochip;
Figure **11** is a view showing another example of a screen for displaying results obtained with the biochip;
Figure **12** is a diagram showing processes of reading and analyzing biochip image/memory information; and
Figure **13** is a flow chart for illustrating processes of reading and analyzing biochip image/memory information.

### BEST MODE FOR CARRYING OUT THE INVENTION

Hereinafter, the present invention will be described in detail with reference to the drawings.

Figure **1** is a schematic view showing an exemplary biochip of the invention. The exemplary biochip **10** is provided with an immobilization substrate **11** (such as a glass, nylon or nitrocellulose membrane) and a storage medium **13**, the substrate **11** being spotted with biopolymers **12** (such as DNAs or proteins) at about 10,000/cm². The surface of the storage medium **13** needs to be covered with plastic, glass or the like or otherwise the storage medium **13** may be exposed, together with the biopolymers **12**, to a sample solution upon hybridization. The biochip **10** is made, for example, with a semiconductor memory support (such as a silicon wafer) as the immobilization substrate **11**; a semiconductor memory formed on part of the support as the storage medium **13**, the top of the memory being covered with a resin or the like; and biopolymers **12** such as DNAs directly spotted onto the remaining surface of the silicon support. This approach allows minimization of the biochip.

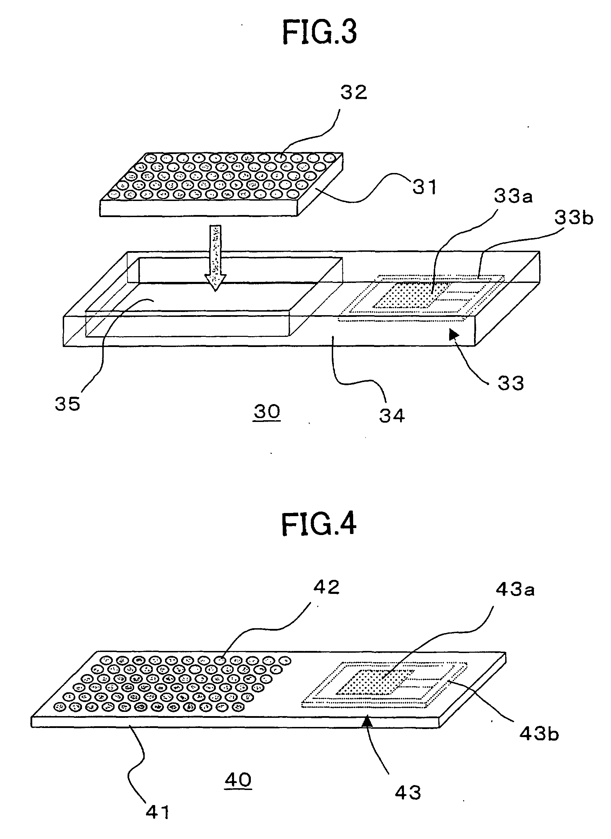

Figure **3** is a schematic view showing another example of the biochip of the invention. This exemplary biochip **30** includes a case **34** and a chip **31** whose surface is spotted with biopolymers **32** such as DNAs. The case **34** has a storage medium **33a** imbedded therein, and has a cavity **35** for accommodating the chip **31**. The storage medium **33a** is an IC memory which is connected to a looped antenna **33b** which is also imbedded in the case **34**, thereby forming a non-contact storage means **33**.

The non-contact storage means **33** receives an electromagnetic wave sent from a reader/writer located close to the biochip **30** via the antenna **33b**. An electromotive force generated by electromagnetic induction is used as electric power for data communication to write/read information to/from the storage medium **33b**. The non-contact storage means **30** reads/writes information in a non-contact state without requiring an externally exposed terminal and thus is completely enclosed and isolated from the external environment. Accordingly, the non-contact storage means **33** is preferable as a memory for a biochip that is exposed to a reagent or a sample solution.

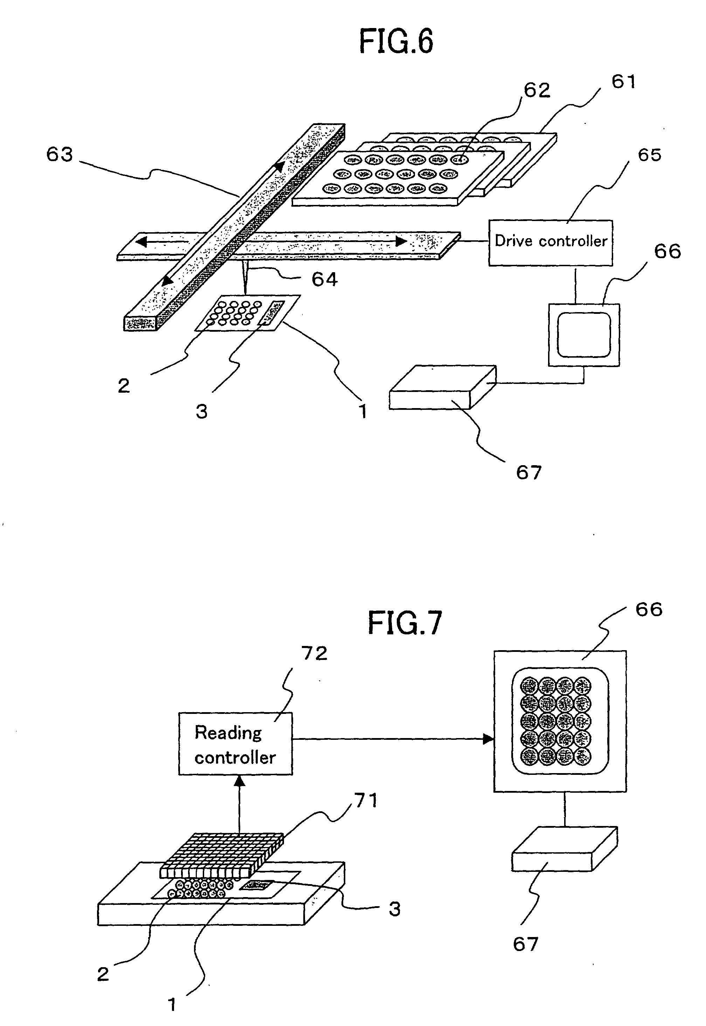

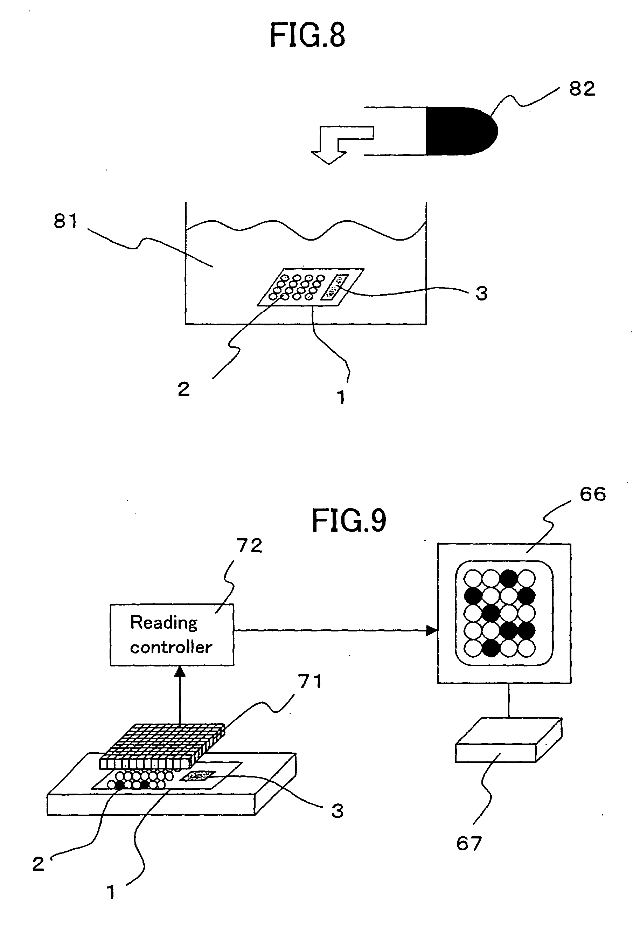

The biochip **30** shown in Figure **3** is used generally as follows. The process for spotting biopolymers onto the biochip **30** is conducted with only the chip **31** separated from the case **34**. Then, the chip **31** is fit into the cavity **35** of the case **34** to form an integral body. The integrated biochip **30** is subjected to a later-described inspection step shown in Figure **7** to write inspection information to the memory medium **33a** of the non-contact storage means **33**. Then, the chip **31** is separated from the case **33** and immersed into a hybridization solution to perform a later-described hybridization reaction step shown in Figure **8**. After the hybridization reaction, the chip **31** is again assembled with the case **34** to perform a later-described reading process shown in Figure **9**. Data obtained through the reading process is stored into the storage medium **33a** of the non-contact storage means **33**.

According to the biochip **30** of a system shown in Figure **3**, the case **34** provided with the non-contact storage means **33** is not exposed to a solution during the hybridization reaction. Accordingly, there is less limitation as to the material used for the case **34**. Since the case **34** is not exposed to a solution, a contact-type storage means whose terminals and the like are exposed above the surface of the case may be used instead of the non-contact storage means **33**. The case **34** may be reused by deleting the stored data in the storage medium **33a**.

Figure **4** is a schematic view showing another example of a biochip of the invention. This exemplary biochip **40** is produced by: partially forming a cavity in an immobilization substrate **41** (such as glass or plastic) by an etching process or the like; placing a non-contact storage means **43** made from a looped antenna **43b** and a storage medium **43a** connected thereto; and imbedding the storage medium **43a** with a resin or the like. The surface of the immobilization substrate **41** is spotted with biopolymers **42** such as DNAs in a predetermined pattern. The biochip of such a system has a simple mechanism as compared to the biochip shown in Figure **3**. Since the storage medium is integrally imbedded in the material to be spotted with the biopolymers, the entire biochip may be minimized.

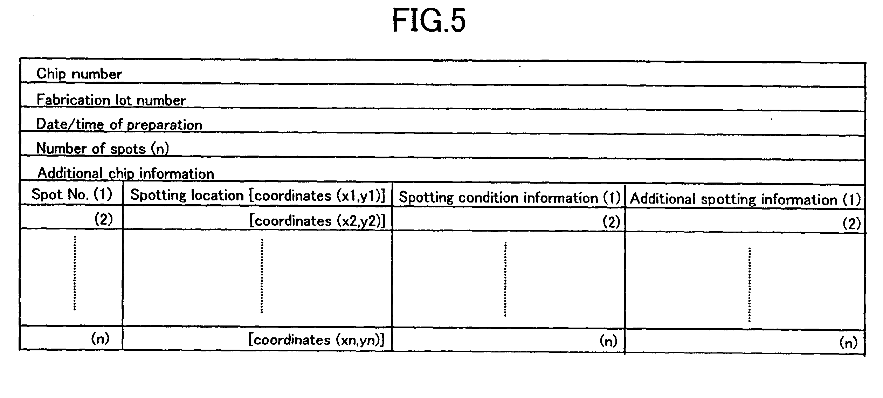

Figure **5** is a diagram showing an example of information to be stored in a storage medium for a single biochip. The information to be stored include general information of the biochip such as a serial number, a fabrication lot number, date/time of preparation, the number of spots on the biochip, and other additional chip information, as well as information of respective spots on the biochip. The information of respective spots on the biochip include, for example, spot numbers, X-Y-coordinates on the biochip, spotting conditions such as amount and time of spotting, and additional information such as names and functions of the spotted DNAs or proteins. The spot numbers are sequentially provided in accordance with the order of spotting. The X-Y coordinates are represented, for example, while the origin is at the upper left corner of the biochip. When the biochip is used for DNA diagnosis, information of an individual and information usually written in a clinical record may also be stored.

A method for managing information in the chip will be described with reference to Figures **6** to **9**. Figure **6** is a view for illustrating process for producing the biochip. A microplate **61** has various biopolymers (for example, sample DNAs **62** having known nucleotide sequences) placed at known respective locations. Under the control of a drive controller **65** controlled by a computer **66**, a pin **64** is transferred to a predetermined location on the microplate 61 with an X-Y driver **63** and made to contact at the tip thereof with the DNA at that location. The pin **64** is again transferred to a predetermined location on the biochip **1** to spot the DNA onto the surface of the chip, thereby forming a DNA spot **2** on the biochip **1**. By repeating this action, each sample DNA 62 on the microplate **61** will be spotted onto the biochip **1** in a predetermined pattern. The biochip **1** is provided with a memory **3**.

The computer **66** commands a reader/writer **67** to write to the memory **3**, spot numbers, spotting locations, nucleic acid sequences of the sample DNAs **62** applied to the spotting locations, name of the genes, the number and location of the microplate **61** and preparation date of the biochip **1**, for example, as in a format shown in Figure **5**. The reader/writer **67** is preferably of a non-contact type, but may be a contact-type for biochips such as one shown in Figure **3**. The information may be written with the reader/writer **67** one at a time synchronous with the spotting operation or at one time after completing the entire spotting manipulation.

Figure **7** is a view for illustrating a step for inspecting the biochip. In this step, the biochip **1** produced through the process shown in Figure **6** is subjected to an inspection of, for example, whether the DNAs are spotted appropriately onto all of the spotting locations **2**. The inspection results are written to the memory **3** of the biochip.

An image of the spot arrangement on the biochip **1** is taken with an image pick-up camera **71** such as a CCD camera and the image data is transferred to the computer **66** via a reading controller **72**. The computer **66** analyzes the image data of the spot arrangement to detect defective spots where an amount of spotted DNA is inadequate. All of the sample DNAs **62** on the microplate **61** may be provided with a fluorescent material such as FITC (fluorescein isothiocyanate), in which case spot points **2** may be irradiated with excitation light from an argon ion laser or the like, so as to detect defective spots based on the presence and absence of the fluorescent light from the fluorescent material at each spotting location. The fluorescent intensity from each spot may also be measured so as to detect the amount of DNA spotted onto each spot. The spot number of the defective spot, the amount of DNA spotted onto each spot may be written to the memory **3** with the reader/writer 67 under the control of the computer **66**.

After the inspection step shown in Figure **7**, the DNA of the defective spot may be spotted again by the spotting step shown in Figure **6**. In this case, the location to be spotted again may be the same as the location found to be defective or may be at an alternative location different from the first spotting location. Furthermore, instead of directly taking the image of the spot arrangement on the biochip, it may be taken via a phase contrast microscope.

Figure **8** is a view for illustrating a hybridization process using the biochip of the invention. As shown in the figure, the biochip **1** provided with the storage medium **3** and the biopolymers **2** such as DNAs, together with fluorescent-labeled sample DNA **82**, is placed into a hybridization solution **81** for hybridization. When there is a complementary DNA sequence between the DNA **2** spotted on the biochip **1** and the sample DNA **82**, the DNAs bind to each other and form a duplex structure on the spot.

Figure **9** is a view for illustrating the reading and analyzing processes of the biochip of the invention. The DNA of the spot **2** on the biochip **1** which has bound with the sample DNA is detected by radiating excitation light to the spots **2** on the biochip **1** and reading the fluorescence emitted from the spot with an optical sensor (such as a CCD **71**). The data read with the optical sensor is transferred to the computer **66** via the reading controller **72**. The computer **66** uses information of the fluorescence location on the biochip **1** read with the optical sensor, and information of the spot read from the memory **3** of the biochip **1** using the reader/writer **67**, thereby deriving information of the sample DNA on the biochip that hybridized with the DNA. Specifically, from the results read with the optical sensor, the information in the memory **3** corresponding to the DNA on the biochip **1** which is suspected to have hybridized is output to the display of the computer **66**.

The data is normalized based on the difference between the data of the amount of the DNA of the spot stored in the memory **3** and the amount of the DNA that hybridized obtained with the optical sensor. The results are written to the memory **3** with the reader/writer **67**. Accordingly, the information can unitarily be managed. In addition, quantitative analyasis and expression level analysis are also possible.

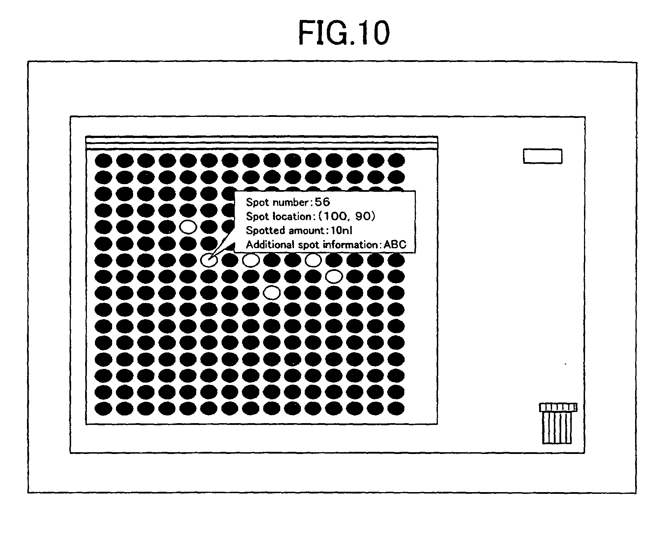

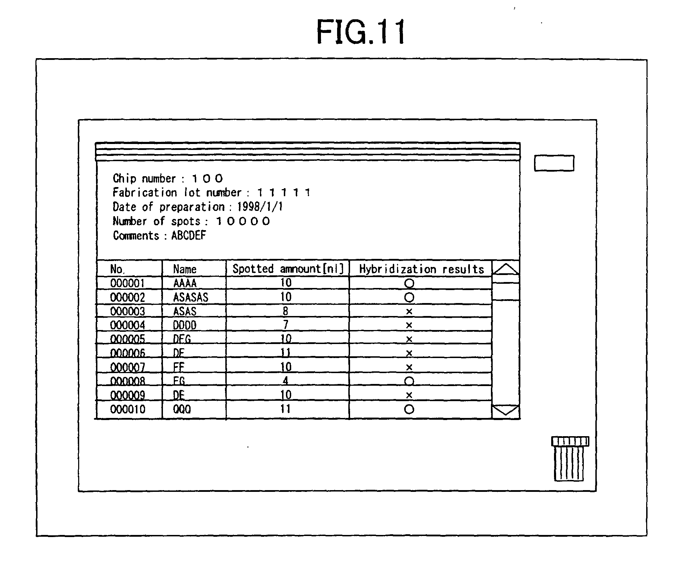

Figures **10** and **11** are views showing exemplary screens displaying analysis results obtained with the biochip as described with reference to Figure **9**. The exemplary screen shown in Figure **10** displays fluorescent intensity image of the biochip read with the optical sensor. When the operator points the image of the spot that (s)he wants to know in detail (e.g., with a mouse cursor), the additional information stored in the memory will be read out with the reader/writer **67** and displayed on the screen.

The exemplary screen shown in Figure **11** displays the analysis results in a list format. Referring to Figure **11**, identification and additional information of the biochip, information of each spot and the results of the hybridization reaction are displayed. The hybridization reaction results are shown as ○ for those that went through hybridization, or × for those that did not cause hybridization. Although information of all of the spots is displayed according to this example, the results may be displayed, after an appropriate filtering process, for example, with a list of only the spots that went through hybridization. The screens shown in Figures **10** and **11** may alternately be displayed from one another.

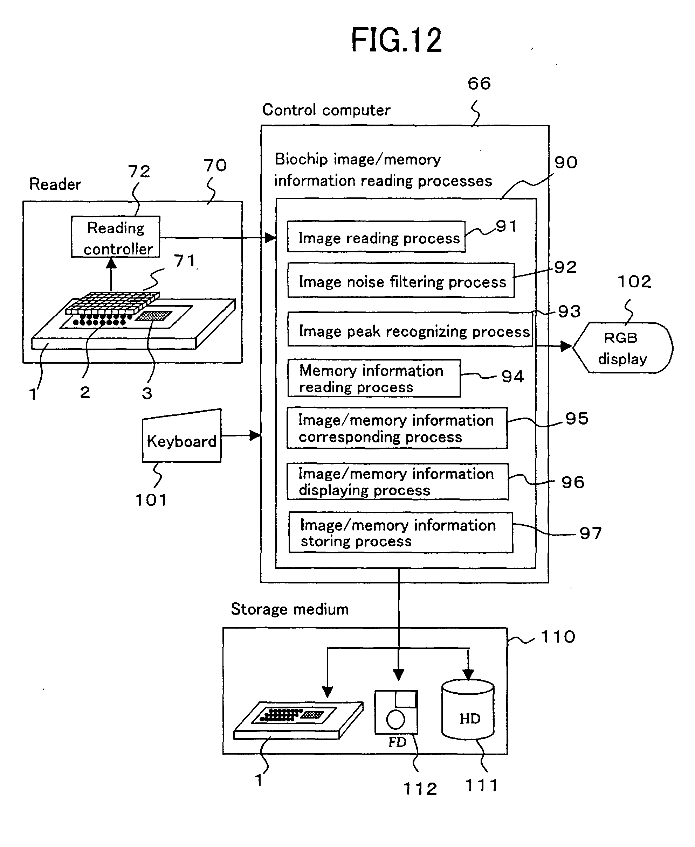

Figure **12** is a diagram illustrating a series of processes for using the biochip. A biochip image/memory information reading program **90** loaded into the computer **66** is initiated as instructed via an input device such as a keyboard **101**. An image reading module **91** of the biochip image/memory information reading program **90** loaded into the computer **66** controls a reading controller **72** of a biochip reader **70** and reads the fluorescent intensities of the spots **2** on the biochip **1** with an optical sensor such as a CCD **71**. The image data read out is transferred from the reading controller **72** to the computer **66**. The transferred image data is subjected to noise elimination and peak recognition processes with a noise filtering module **92** and an image peak recognizing module **93**, respectively, thereby determining the peak coordinates and intensity of the fluorescence at each spot.

Then, the memory information reading module **94** instructs the biochip reader **70** to read spot information stored in the memory **3** on the biochip **1**. The spot information read out is transferred to the computer **66**. An image/memory information corresponding module **95** corresponds the spot information with information of the image data, peak coordinates and fluorescent intensity. An image/memory information screen displaying module **96** displays the corresponded image/memory information on an RGB display **102**. An image/memory information storing module **97** stores the image/memory information in a storage medium **110** such as a hard disk device **111**, a floppy disk device **112** or the biochip **1**. Each of the modules shown in Figure **12** may be realized by a software program.

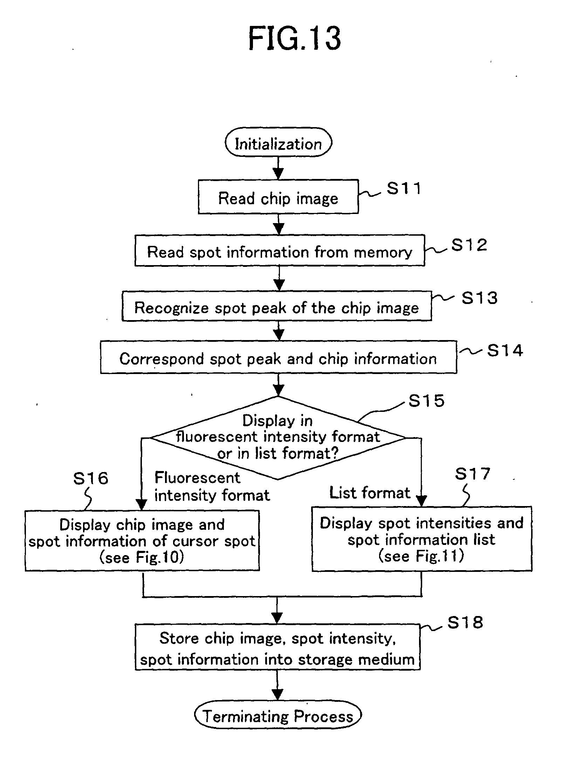

Figure **13** is a flow chart for illustrating processes of reading and analyzing biochip image/memory information. The reading device and the like are initialized, followed by chip image reading process (**S11**) for reading fluorescent intensities of spots **2** on the biochip **1** with the optical sensor such as the CCD **71**; process (**S12**) for reading spot information stored in memory **3** of the biochip **1**; process (**S13**) for recognizing peak coordinates and peak intensities of the fluorescence of the spots of the chip image; and process (**S14**) for corresponding the spot peak information obtained from the chip image and the spot information stored in the memory. The information obtained through such a series of process, according to the selected display format at **S15**, is subjected to process for displaying the chip image and information of a spot at a cursor point (**S16**) or to process for displaying the list of spot intensities and spot information (**S17**). The chip image, spot intensities and spot information are stored in a storage medium such as the hard disc device **111**, the floppy disc device **112** or the biochip **1**. Thus, a terminating process terminates the reading/analyzing process of the biochip image/memory information.

The biochip of the invention provided with a memory allows unitary management of information by storing data of, for example, a sample DNA and experimental environment used for the biochip, as well as the results of an analysis or the like in the memory. Accordingly, an experimental error can be avoided. Moreover, accurate experimental results can be obtained if information of an amount of each of the DNAs on the chip is stored in the memory in advance such that the results are obtained based on the difference from the amount of DNA upon chip reading after the hybridization experiment or the like. In the case where the results of the analysis are managed as a database, the information may directly be read from the memory so that information may be managed in a facilitated manner.

### INDUSTRIAL APPLICATION

According to the present invention, unitary management of information is realized by storing information of a biochip in the biochip itself, thereby preventing errors and realizing rapid and accurate process.

## Claims

1. A biochip comprising
a surface spotted with a plurality of biopolymers in predetermined pattern,
a storage medium for storing information of the biopolymers to be spotted, and
a looped antenna,
the storage medium being an IC memory connected to the looped antenna, the storage medium thereby being capable of reading/writing information in a non-contact state, the biochip having a plurality of biopolymers spotted thereon, wherein the storage medium stores information of the spot locations, on the identity of the biopolymers spotted on each spot location and on the amount of biopolymers spotted on each spot location.

2. The biochip according to claim 1, wherein a member provided with the surface and the storage medium are detachable, from each other.

3. The biochip according to claim 1, wherein a member provided with the surface and the storage medium are formed integrally.

4. The biochip according to any of the foregoing claims, having about 10,000 spots/cm².

5. A method of manufacturing a biochip according to any of the foregoing claims, comprising the steps:
- spotting a plurality of biopolymers on a surface of the biochip in a predetermined pattern,
- writing information of the spot locations to the storage medium in relation to the information of biopolymers spotted on the spot locations.

6. Use of a biochip manufactured according to claim 5, comprising the steps
- applying a sample to the biochip,
- detecting a spot location where hybridization has occurred,
- searching the storage medium for information on the biopolymer that has hybridized, the search being based on information about the spot location where hybridization has occurred,
- displaying the information on the biopolymer that has hybridized.

## Patentansprüche

1. Biochip, umfassend
eine Oberfläche, die mit einer Vielzahl von Biopolymeren in einem vorbestimmten Muster betupft ist,
ein Speichermedium zum Speichern von Information im Hinblick auf die aufzutupfenden Biopolymere, und
eine schleifenförmigen Antenne,
wobei das Speichermedium ein IC-Speicher ist, der mit der schleifenförmigen Antenne verbunden ist, wobei das Speichermedium dadurch in der Lage ist, Information in einem kontaktlosen Zustand zu lesen/zu schreiben, wobei der Biochip eine Vielzahl von aufgetupften Biopolymeren hat, wobei das Speichermedium Information im Hinblick auf die Tupfenpositionen, im Hinblick auf die Identität der Biopolymere, die auf jeder Tupfenposition aufgetupft sind und im Hinblick auf die Menge der Biopolymere, die an jeder Tupfenposition aufgetupft sind, speichert.

2. Biochip nach Anspruch 1, wobei ein Teil, das mit der Oberfläche bereitgestellt wird, und das Speichermedium voneinander lösbar sind.

3. Biochip nach Anspruch 1, wobei ein Teil, das mit der Oberfläche bereitgestellt wird, und das Speichermedium miteinander integriert gebildet sind.

4. Biochip nach einem der vorangehenden Ansprüche mit ungefähr 10.000 Tupfen/cm².

5. Verfahren zum Herstellen eines Biochip nach einem der vorangehenden Ansprüche, umfassend die Schritte:
- Auftupfen einer Vielzahl von Biopolymeren auf einer Oberfläche des Biochip in einem vorbestimmten Muster,
- Schreiben von Information auf das Speichermedium über die Tupfenposition in Bezug auf die Information über die Biopolymere, die auf die Tupfenpositionen aufgetupft sind.

6. Verwendung eines Biochip, hergestellt nach Anspruch 5, umfassend die Schritte
- Aufbringen einer Probe auf den Biochip,
- Nachweisen einer Tupfenposition, an der eine Hybridisierung stattgefunden hat,
- Suchen in dem Speichermedium nach Information im Hinblick auf das Biopolymer, das hybridisiert hat, wobei die Suche auf Information über die Tupfenposition, an der eine Hybridisierung stattgefunden hat, beruht,
- Darstellen der Information über das Biopolymer, das hybridisiert hat.

## Revendications

1. Biopuce comprenant
une surface sur laquelle ont été greffés plusieurs biopolymères selon un agencement prédéterminé,
une mémoire pour mémoriser des informations sur les biopolymères à greffer, et
une antenne en boucle,
la mémoire étant une mémoire intégrée connectée à l'antenne en boucle, la mémoire pouvant ainsi lire/écrire des informations dans un état sans contact, la biopuce comportant une pluralité de biopolymères greffés sur sa surface, la mémoire mémorisant des informations sur les emplacements de greffage, sur l'identité des biopolymères greffés à chaque emplacement de greffage et sur la quantité de biopolymères greffés à chaque emplacement de greffage.

2. Biopuce selon la revendication 1, dans laquelle un élément comportant ladite surface et la mémoire sont séparables l'un de l'autre.

3. Biopuce selon la revendication 1, dans laquelle un élément comportant ladite surface et la mémoire sont solidaires.

4. Biopuce selon l'une quelconque des revendications précédentes, comportant environ 10000 sites de greffage/cm².

5. Procédé de fabrication d'une biopuce selon l'une quelconque des revendications précédentes, comprenant les étapes suivantes :
- greffer une pluralité de biopolymères sur une surface de la biopuce selon un agencement prédéterminé,
- écrire des informations sur les emplacements de greffage dans la mémoire en relation avec les informations concernant des biopolymères greffés sur les emplacements de greffage.

6. Utilisation d'une biopuce fabriquée selon la revendication 5, comprenant les étapes suivantes :
- appliquer un échantillon sur la biopuce,
- détecter un emplacement de greffage où une hybridation a eu lieu,
- rechercher dans la mémoire des informations sur le biopolymère qui s'est hybridé, la recherche étant fondée sur des informations concernant l'emplacement de greffage où une hybridation a eu lieu,
- afficher les informations concernant le biopolymère qui s'est hybridé.
